# EUROPÄISCHE PATENTSCHRIFT

(11) **EP 4 193 945 B1**
(45) Veröffentlichungstag und Bekanntmachung des Hinweises auf die Patenterteilung: **05.11.2025**
(21) Anmeldenummer: 22210541.3
(22) Anmeldetag: 30.11.2022
(51) Int. Cl.: A61B 18/14

(54) **VERBINDUNGSVORRICHTUNG FÜR CHIRURGISCHE INSTRUMENTE SOWIE CHIRURGISCHES INSTRUMENT UND VERFAHREN**
CONNECTING DEVICE FOR SURGICAL INSTRUMENTS, SURGICAL INSTRUMENT AND METHOD
DISPOSITIF DE CONNEXION POUR INSTRUMENTS CHIRURGICAUX, INSTRUMENT CHIRURGICAL ET PROCÉDÉ

(30) Priorität: 09.12.2021 DE 102021132426
(43) Veröffentlichungstag der Anmeldung: 14.06.2023
(73) Patentinhaber: Karl Storz SE & Co. KG, 78532 Tuttlingen (DE)
(72) Erfinder: PREIS, Fridolin, 78532 Tuttlingen (DE)

(56) Entgegenhaltungen:
- EP-A2- 2 140 803
- WO-A2-2016/138443
- WO-A2-2017/035221
- GB-A- 2 567 161
- RU-U1- 206 061
- US-A- 5 971 940
- US-A1- 2011 295 066

## Beschreibung

### GEBIET DER ERFINDUNG

Die vorliegende Erfindung betrifft eine Verbindungsvorrichtung für chirurgische Instrumente, sowie ein chirurgisches Instrument und ein Verfahren.

### TECHNISCHER HINTERGRUND

Chirurgische Instrumente weisen oftmals eine Verbindungsvorrichtung und ein daran mittels einer Kopfteilkupplung anbringbares Führungselement für ein Zubehör auf. Das Führungselement bildet ein Kopfteil aus, das an einem distalen Ende des chirurgischen Instruments angeordnet ist.

In der HF-Chirurgie wird bei der sogenannten Elektrotomie (auch Diathermie oder Elektrokaustik) und der Koagulation (Gerinnung) die thermische Wirkung von hochfrequentem Wechselstrom auf Gewebe ausgenutzt, um Zielgewebe zu durchtrennen und gegebenenfalls gleichzeitig oder stattdessen Blutungen zu stillen. Die chirurgischen Instrumente weisen dazu eine oder mehrere (Aktiv-) Elektroden an ihrem Zubehör auf, über welche der hochfrequente Wechselstrom in das Zielgewebe eines Patienten eingeleitet wird. Damit am Zielgewebe der gewünschte Effekt der Elektrotomie und/oder Koagulation auftritt, wird eine hohe Stromdichte am Zielgewebe eingebracht. Daher weisen (Aktiv-) Elektroden in der Regel eine geringe Oberfläche auf und sind dazu beispielsweise nadelförmig oder klingenförmig ausgebildet, um den angelegten hochfrequenten Wechselstrom mit möglichst hoher Stromdichte in das Zielgewebe einzubringen. Es können dabei entweder monopolar oder bipolar betriebene chirurgische Instrumente zum Einsatz kommen.

Bei monopolar betriebenen chirurgischen Instrumenten ist an dem Zubehör des chirurgischen Instruments eine einzige Aktivelektrode angeordnet, über welche der hochfrequente Wechselstrom in das Gewebe eines Patienten eingeleitet wird. Damit von der einzelnen Aktivelektrode aus der Strom in das Gewebe eingeleitet werden kann, muss eine großflächige Gegenelektrode (Neutralelektrode) an dem Körper des Patienten als Gegenpol angebracht werden.

Bei bipolar betriebenen chirurgischen Instrumenten sind an dem Zubehör des chirurgischen Instruments zwei Elektroden (Aktivelektrode und Neutralelektrode) angeordnet. Dabei wird von einer ersten Elektrode (Aktivelektrode) aus der hochfrequenter Wechselstrom direkt gegenüber der zweiten Elektrode (Neutralelektrode) in das Zielgewebe eingebracht.

Ein monopolares oder bipolares chirurgisches Instrument kann als Resektoskop ausgebildet sein, insbesondere zur transurethralen Resektion. Bei einem bipolaren Resektoskop muss ein optimiertes Anschnittverhalten und eine präzise Schnitt- bzw. Koagulationsqualität während des gesamten Eingriffs sichergestellt sein.

Das Kopfteil, insbesondere bei einem Resektoskop, ist an einem Schaft des chirurgischen Instruments angeordnet und bildet das frontale bzw. distale Element aus, das zur Führung des chirurgischen Instruments durch einen Körperabschnitt dient. Dafür muss das Kopfteil ausreichend stabil und langlebig ausgebildet sein. Durch das Kopfteil kann verschiedenartiges Zubehör, beispielsweise eine kalte Schlinge, ein diagnostisches Hysteroskop oder HF-Elektroden für unterschiedliche Indikatoren gehalten werden. Da das Kopfteil direkt an der durch eine Kraft beanspruchten Stelle angeordnet ist, muss dieses stabil an dem Schaft befestigt werden. Eine Fixierung des Kopfteils an dem Schaft kann beispielsweise durch eine Klebeverbindung vorgesehen sein.

EP2140803A2, GB2567161A und RU206061U1 offenbaren chirurgische Instrumente des Standes der Technik.

### ZUSAMMENFASSUNG DER ERFINDUNG

Vor diesem Hintergrund liegt der vorliegenden Erfindung die Aufgabe zugrunde, eine verbesserte Verbindungsvorrichtung für chirurgische Instrumente, insbesondere bipolar betreibbare chirurgische Instrumente, bereitzustellen.

Erfindungsgemäß wird diese Aufgabe durch eine Verbindungsvorrichtung mit den Merkmalen des unabhängigen Patentanspruchs 1 sowie ein chirurgisches Instrument und ein Verfahren mit den Merkmalen der weiteren unabhängigen Patentansprüche gelöst.

Demgemäß ist vorgesehen:
- Eine Verbindungsvorrichtung für chirurgische Instrumente, mit einem Gehäuse mit einem Schaft, einem Kopfteil und mit einer Kopfteilkupplung, die an dem Gehäuse angeordnet und zum mechanischen Koppeln des Kopfteils ausgebildet ist, wobei das Kopfteil an einem distalen Ende des Schafts angeordnet ist, und wobei durch das Kopfteil ein Zubehör durchführbar ist, wobei die Kopfteilkupplung ein Stiftelement aufweist, das derart ausgebildet ist, dass das Kopfteil mit dem Schaft des Gehäuses unverschieblich fixierbar ist, wobei das Stiftelement mit dem Schaft des Gehäuses über eine Schweißnaht unlösbar fixiert ist.
- Ein chirurgisches Instrument, insbesondere für die Hochfrequenzchirurgie, mit einer erfindungsgemäßen Verbindungsvorrichtung.
- Ein Verfahren zur Fixierung einer erfindungsgemäßen Verbindungsvorrichtung, wobei das Stiftelement mittels eines Laserschweißverfahrens an dem Schaft des Gehäuses unlösbar fixiert wird.

Die der vorliegenden Erfindung zugrundeliegende Idee besteht darin, durch eine formschlüssige Verbindung in Kombination mit einer Schweißnaht das Kopfteil unlösbar und unverlierbar an dem Gehäuse, insbesondere an einem Schaft des Instruments, zu fixieren.

Das chirurgische Instrument kann insbesondere als monopolar und/oder bipolar betreibbares chirurgisches Instrument ausgebildet sein. Insbesondere in der Hochfrequenzchirurgie kann mit dem wahlweise monopolar oder bipolar betreibbaren chirurgischen Instrument Gewebe durchtrennt werden (Elektrotomie) sowie zusätzlich oder alternativ Blutungen gestillt werden (Koagulation). Das monopolar und bipolar betreibbare chirurgische Instrument kann beispielsweise ein durch einen Nutzer bzw. Bediener (z. B. Chirurg) von Hand oder ein durch einen Roboter(-arm) geführtes Instrument sein.

Das Gehäuse kann integral bzw. einstückig ausgeführt sein oder aus mehreren Komponenten (z. B. Schalenelementen), die mit einander lösbar oder fest mechanisch verbunden sind, gebildet sein. Das Gehäuse kann zumindest teilweise aus einem Kunststoff, einem Metall oder einer Legierung gefertigt sein. Bevorzugt kann das Gehäuse zumindest teilweise aus einem biokompatiblen Werkstoff gefertigt sein.

Das Gehäuse weist einen Schaft auf, der über die Kopfteilkupplung mit dem Kopfteil unlösbar und sicher fixiert ist.

Das Gehäuse ist insbesondere zumindest in dem Bereich, in dem es mit dem Kopfteil in Verbindung steht, rohrförmig ausgebildet und kann insbesondere einen Durchmesser von 4 mm bis 10 mm, beispielsweise 5 mm, aufweisen.

Der Schaft ist bevorzugt zur zumindest teilweisen Aufnahme des Kopfteils ausgebildet. Beispielsweise kann der Schaft eine Art Außenrohr für das Kopfteil ausbilden. Insbesondere kann das Kopfteil mit einem Teilabschnitt in den Schaft eingesteckt werden, um das Kopfteil auszurichten und an die geometrisch gewollte Position zu bringen. Über die Verbindungsvorrichtung erfolgt daher insbesondere die unlösbare Fixierung des Kopfteils an dem Schaft. Die Verbindungsvorrichtung ist derart ausgebildet, dass ein damit mechanisch verbundenes Kopfteil unverlierbar gehalten werden kann.

Die Kopfteilkupplung weist ein Stiftelement auf, das eine formschlüssige Verbindung zwischen dem Gehäuse, insbesondere dem Schaft, und dem Kopfteil ausbildet. Insbesondere ist das Stiftelement klein im Verhältnis zu einem Durchmesser des Kopfteils bzw. des Gehäuses ausgebildet. Das Kopfteil kann zur Führung eines Zubehörs ausgebildet sein.

Das Kopfteil ist insbesondere ein Keramikeinsatz, der an einem distalen Ende des Schafts eingesteckt und über das Stiftelement formschlüssig fixiert wird.

Die Schweißnaht ist bevorzugt durch ein Laserschweißverfahren hergestellt, sodass eine präzise und für einen gewünschten Toleranzbereich notwendige Schweißnaht ausgebildet werden kann.

Mit der erfindungsgemäßen Verbindungsvorrichtung kann schnell und aufwandsarm das Kopfteil an dem Gehäuse unlösbar fixiert werden, sodass ein Herausfallen des Kopfteils während der OP, und ein Verbleib im Patienten, sicher vermieden werden kann.

Vorteilhafte Ausgestaltungen und Weiterbildungen ergeben sich aus den weiteren Unteransprüchen sowie aus der Beschreibung unter Bezugnahme auf die Figuren der Zeichnung.

Gemäß einer vorteilhaften Ausführungsform kann das Stiftelement durch eine Bohrung durch das Gehäuse hindurchgreifen und in eine Ausformung in dem Kopfteil eingreifen. Vorteilhaft ist ein Durchmesser der Bohrung an einen Durchmesser des Stiftelements angepasst, sodass das Stiftelement sicher an dem Gehäuse verschweißt werden kann. Insbesondere schließt das Stiftelement bündig mit einer Außenoberfläche des Gehäuses ab, sodass das chirurgische Instrument durch die Verbindungsvorrichtung nicht beeinflusst wird. Die Ausformung in dem Kopfteil kann als Senke bzw. topförmige Aufnahme ausgebildet sein. Das Stiftelement wird daher bevorzugt innerhalb der Ausformung gehalten und kann das Kopfteil nicht durchstoßen.

Gemäß einer Weiterbildung kann das Stiftelement das Kopfteil mit dem Gehäuse formschlüssig verbinden. Auf diese Weise ist ein Verschieben des Kopfteils relativ zu dem Gehäuse unterbunden, wodurch das Kopfteil lagefixiert gehalten werden kann.

Gemäß einer Ausführungsform kann das Gehäuse rohrförmig ausgebildet sein. Vorteilhaft ist auf diese Weise ein zumindest teilweises Einstecken des Kopfteils in das Gehäuse möglich. Das Gehäuse bildet bevorzugt einen Schaft aus, der einen Durchmesser von beispielsweise 5 mm bis 10 mm aufweisen kann. Weiterhin sind Durchmesser denkbar, die kleiner als 5 mm betragen. Die zur Verfügung stehende Wanddicke des rohrförmigen Gehäuses ist daher sehr gering, sodass ein Verschweißen des Stiftelements mit dem Gehäuse aufgrund der geringen Toleranzschwelle bevorzugt durch Laserschweißen erfolgt.

Gemäß einer vorteilhaften Ausführungsform kann das Kopfteil rohrförmig ausgebildet sein. In einer derartigen Ausführungsform ist vorteilhafterweise das Gehäuse ebenso rohrförmige ausgebildet. Das Gehäuse kann dabei eine Art Außenrohr ausbilden, in das ein Teilbereich des Kopfteils eingesteckt wird. Die Durchmesser von Kopfteil und Außenrohr sind bevorzugt aneinander angepasst, sodass nur ein geringer Spalt entsteht.

Erfindungsgemäß ist durch das Kopfteil ein Zubehör durchführbar. Insbesondere kann ein monopolar und/oder bipolar betreibbares Zubehör durchführbar sein. Dies wird insbesondere dadurch ermöglicht, dass das Kopfteil einen Hohlquerschnitt ausbildet. Die Kopfteilkupplung, d. h. insbesondere das Stiftelement, beeinflusst den Bereich, der zur Durchführung des Zubehörs dient, nicht.

Gemäß einer bevorzugten Ausführungsform kann das Stiftelement quer zu einer Längsachse des Gehäuses ausgerichtet sein. Vorteilhaft ist auf diese Weise eine formschlüssige Verbindung möglich, da das Stiftelement quer zu einer Belastungsrichtung bei einem Einführen bzw. bei einem Herausnehmen des chirurgischen Instruments in bzw. aus der Körperregion ausgerichtet ist.

Gemäß einer bevorzugten Ausführungsform kann das Kopfteil Keramik enthalten oder aus Keramik ausgebildet sein. Auf diese Weise ist die Ausführung eines besonders stabilen sowie langlebigen Kopfteils möglich. Insbesondere können thermische Schäden am Kopfteil durch den Einsatz einer Keramik verhindert werden.

Gemäß einer besonders bevorzugten Ausführungsform kann die Verbindungsvorrichtung für ein Resektoskop ausgebildet sein. Insbesondere kann es sich um ein 5-mm-Resektoskop handeln. Ein derartiges Resektoskop bietet aufgrund des sehr geringen Durchmessers die Möglichkeit, ambulant Infertilitätspatientinnen sowie Patienten mit Blutungsstörungen ohne Anästhesie zu behandeln. Dafür ist nur eine sehr geringe oder keine Dilatation notwendig. Insbesondere kann ein bipolares System ohne Rückführung des Stromflusses über den Schaft, d. h. über das Gehäuse, bereitgestellt werden. Das Kopfteil ist bevorzugt als Keramikeinsatz ausgebildet, und kann beispielsweise für kalte Schlingen, als diagnostisches Hysteroskop sowie für HF-Elektroden für unterschiedliche Indikatoren verwendet werden. Dabei kann eine effektive Resektion, Koagulation oder Vaporisation erreicht werden.

Für das erfindungsgemäße chirurgische Instrument sowie das Verfahren zur Fixierung der Verbindungsvorrichtung gelten dieselben Merkmale und Vorteile, wie bezüglich der Verbindungsvorrichtung beschrieben, sodass im Einzelnen nicht darauf eingegangen wird.

Die obigen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmale der Erfindung. Insbesondere wird dabei der Fachmann auch Einzelaspekte als Verbesserungen oder Ergänzungen zu der jeweiligen Grundform der vorliegenden Erfindung hinzufügen.

### INHALTSANGABE DER ZEICHNUNG

Die vorliegende Erfindung wird nachfolgend anhand der in den schematischen Figuren der Zeichnung angegebenen Ausführungsbeispiele näher erläutert. Es zeigen dabei:
- Fig. 1: ein Ausführungsbeispiel eines chirurgischen Instruments mit einem Kopfteil und einem darin geführten Zubehör;
- Fig. 2: ein Ausführungsbeispiel eines chirurgischen Instruments beim Verschweißen der Kopfteilkupplung.

Die beiliegenden Figuren der Zeichnung sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander gezeigt.

In den Figuren der Zeichnung sind gleiche, funktionsgleiche und gleich wirkende Elemente, Merkmale und Komponenten - sofern nichts Anderes ausgeführt ist - jeweils mit denselben Bezugszeichen versehen.

### BESCHREIBUNG VON AUSFÜHRUNGSBEISPIELEN

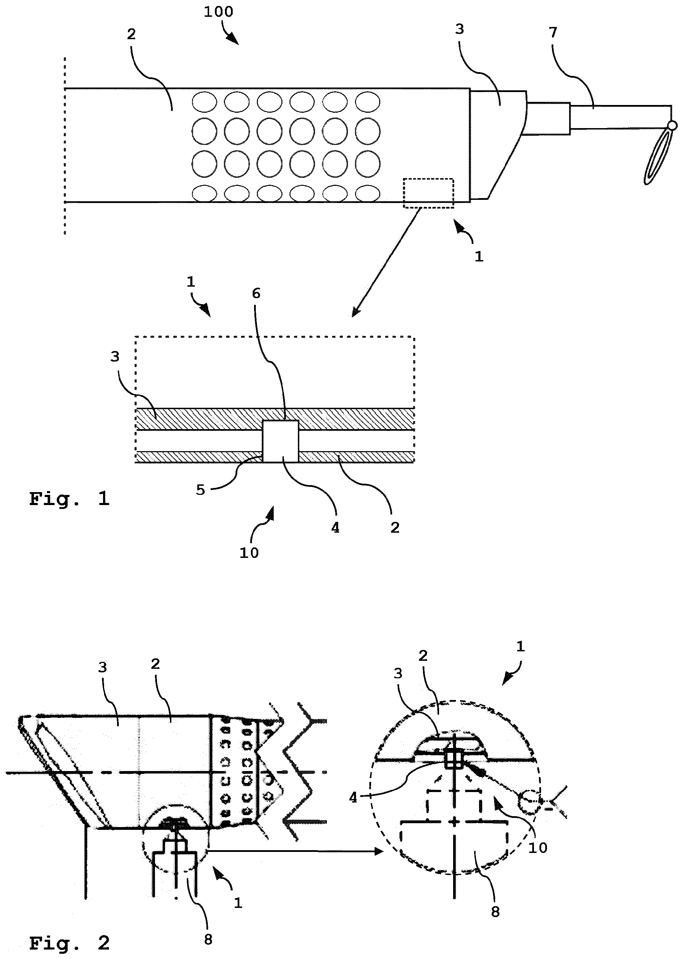

In Fig. 1 ist ein Ausführungsbeispiel eines chirurgischen Instruments 100 mit einem Kopfteil 3 und einem darin geführten Zubehör 7 dargestellt. Von dem chirurgischen Instrument 100 ist lediglich ein Frontbereich dargestellt, in welchem ein Teilbereich des Gehäuses 2 zu sehen ist. Die Kopfteilkupplung 10 ist an einem distalen Ende des chirurgischen Instruments 100 bzw. des Gehäuses 2 angeordnet. Die Kopfteilkupplung 10 ist zum unlösbaren mechanischen Verbinden des Kopfteils 3 mit dem Gehäuse 2 ausgebildet.

Das Gehäuse 2 bildet einen Schaft aus, der zur Aufnahme eines Kopfteils 3 ausgebildet ist. Durch das Gehäuse 2 sowie durch das Kopfteil 3 ist ein Zubehör 7 geführt, das an dem distalen Ende über das Kopfteil 3 übersteht. In der Darstellung ist das Zubehör 7 als kalte Schlinge ausgeführt. Es ist ebenso denkbar, als Zubehör 7 ein diagnostisches Hysteroskop, optional mit 5 Charr.-Instrumenten, oder ein HF-Elektroden für unterschiedliche Indikatoren, sowie anderweitige Instrumente, anzuordnen.

Das Zubehör 7 ist hier ein bipolar betreibbares Zubehör. Es kann selbstverständlich auch ein monopolar oder ein monopolar und bipolar betreibbares Zubehör an das chirurgische Instrument 100 angeschlossen werden.

Das Kopfteil 3 ist insbesondere als Keramikeinsatz ausgebildet und dadurch formstabil und langlebig.

Die Verbindungsvorrichtung 1 ist als vergrößerter Ausschnitt im Detail dargestellt. Der vergrößerte Ausschnitt zeigt eine Schnittdarstellung, sodass das Eingreifen des Kopfteils 3 in das Gehäuse 2 erkennbar ist. Die Kopfteilkupplung 10 weist ein Stiftelement 4 auf, das eine formschlüssige Verbindung zwischen dem Gehäuse 2 und dem Kopfteil 3 ausbildet. Das Stiftelement 4 ist durch eine Bohrung 5 in dem Gehäuse 2 geführt und wird in einer Ausformung 6 in dem Kopfteil 3 gehalten. Dadurch kann das Stiftelement 4 das Kopfteil 3 nicht durchstoßen. Das Stiftelement 4 ist daher in einer radialen Richtung unverschieblich gehalten.

Das Stiftelement 4 bildet insbesondere eine lokale, mit anderen Worten punktförmige, Fixierung aus und benötigt dadurch wenig Bauraum. Das Stiftelement 4 ist quer zu einer Längsachse des Gehäuses 2 orientiert. Insbesondere wird der Bereich innerhalb des als Hohlquerschnitt ausgebildeten Kopfteils 3 durch das Stiftelement 4 nicht beeinflusst. Weiterhin schließt das Stiftelement 4 bündig mit einer Außenoberfläche des Gehäuses 2 ab.

Das Gehäuse 2 ist in einem Frontbereich des chirurgischen Instruments 100 rohrförmig ausgebildet. Ebenso ist das Kopfteil 3 rohrförmig ausgebildet, wobei ein Durchmesser des Kopfteils 3 oder des Gehäuses 2 in einem Bereich von 5 mm liegen kann. Daher ist ein sehr präzises Schweißverfahren zur Ausführung einer Schweißnaht erforderlich, die das Stiftelement 4 an dem Gehäuse 2 fixiert.

Ein Schweißwerkzeug 8 zur Ausführung einer derartigen Schweißnaht ist in Fig. 2 dargestellt. Die Schweißnaht wird bevorzugt durch ein Laserschweißverfahren ausgeführt, sodass sehr geringe Toleranzen umgesetzt werden können. Die Schweißnaht kann als umlaufende Naht ausgeführt sein. Dabei kann beispielsweise eine V-Naht, eine HV-Naht oder eine Y-Naht ausgebildet werden. Andere Schweißnahtformen sind ebenso denkbar.

Durch den Formschluss zwischen dem Kopfteil 3 und dem Gehäuse 2 in Verbindung mit der Schweißnaht kann ein Lösen des Kopfteils 3 und ein Verbleiben in einem Patienten effektiv vermieden werden, insbesondere im Vergleich zu einer Klebeverbindung aus dem Stand der Technik. Weiterhin ist die Verbindungsvorrichtung 1 kostengünstig und schnell ausführbar, und damit ebenso kostengünstig im Vergleich zu einer Verprägung beider Bauteile.

Obwohl die vorliegende Erfindung anhand bevorzugter Ausführungsbeispiele vorstehend vollständig beschrieben wurde, ist sie darauf nicht beschränkt, sondern auf vielfältige Art und Weise modifizierbar.

Beispielsweise kann das Kopfteil 3 auch außerhalb des Gehäuses 2 geführt sein. In einer derartigen Ausführungsform wäre die Bohrung 5 in dem Kopfteil 3 und die Ausformung 6 in dem Gehäuse 2 ausgeformt. In einer weiteren nicht dargestellten Ausführungsform kann die Verbindungsvorrichtung 1 zwei oder mehr Kopfteilkupplungen 10 aufweisen, die entlang des Umfangs des Gehäuses 2 bzw. des Kopfteils 3 in dem Frontbereich des chirurgischen Instruments 100 angeordnet sind. Dadurch kann die Stabilität der Verbindung weiter gesteigert werden.

### BEZUGSZEICHENLISTE

- 1: Verbindungsvorrichtung
- 2: Gehäuse
- 3: Kopfteil
- 4: Stiftelement
- 5: Bohrung
- 6: Ausformung
- 7: Zubehör
- 8: Schweißwerkzeug

- 10: Kopfteilkopplung
- 100: chirurgisches Instrument

## Patentansprüche

1. Verbindungsvorrichtung (1) für chirurgische Instrumente (100), mit:
einem Gehäuse (2) mit einem Schaft;
einem Kopfteil; und
einer Kopfteilkupplung (10), die an dem Schaft des Gehäuses (2) angeordnet und zum mechanischen Koppeln des Kopfteils (3) ausgebildet ist, wobei das Kopfteil (3) an einem distalen Ende des Schafts angeordnet ist und wobei durch das Kopfteil (3) ein Zubehör (7) durchführbar ist;
wobei die Kopfteilkupplung (10) ein Stiftelement (4) aufweist, das derart ausgebildet ist, dass das Kopfteil (3) mit dem Schaft des Gehäuses (2) unverschieblich fixierbar ist,
**dadurch gekennzeichnet, dass** das Stiftelement (4) mit dem Schaft des Gehäuses (2) über eine Schweißnaht unlösbar fixiert ist.

2. Verbindungsvorrichtung (1) nach Anspruch 1,
**dadurch gekennzeichnet,**
**dass** das Stiftelement (4) durch eine Bohrung (5) durch das Gehäuse (2) hindurchgreift und in eine Ausformung (6) in dem Kopfteil (3) eingreift.

3. Verbindungsvorrichtung (1) nach Anspruch 1 oder 2,
**dadurch gekennzeichnet,**
**dass** das Stiftelement (4) das Kopfteil (3) mit dem Gehäuse (2) formschlüssig verbindet.

4. Verbindungsvorrichtung (1) nach einem der vorstehenden Ansprüche,
**dadurch gekennzeichnet,**
**dass** das Gehäuse (2) und/oder das Kopfteil (3) rohrförmig ausgebildet ist.

5. Verbindungsvorrichtung (1) nach einem der vorstehenden Ansprüche,
**dadurch gekennzeichnet,**
**dass** durch das Kopfteil (3) ein monopolar und/oder bipolar betreibbares Zubehör (7) durchführbar ist.

6. Verbindungsvorrichtung (1) nach einem der vorstehenden Ansprüche,
**dadurch gekennzeichnet,**
**dass** das Stiftelement (4) quer zu einer Längsachse des Gehäuses (2) ausgerichtet ist.

7. Verbindungsvorrichtung (1) nach einem der vorstehenden Ansprüche,
**dadurch gekennzeichnet,**
**dass** das Kopfteil (3) Keramik enthält oder aus Keramik ausgebildet ist.

8. Verbindungsvorrichtung (1) nach einem der vorstehenden Ansprüche,
**dadurch gekennzeichnet,**
**dass** diese für ein Resektoskop ausgebildet ist.

9. Chirurgisches Instrument (100), insbesondere für die Hochfrequenzchirurgie, mit:
einer Verbindungsvorrichtung (1) nach einem der Ansprüche 1 bis 8; und
einem Zubehör (7), insbesondere einem monopolar und/oder bipolar betreibbaren Zubehör (7), welches durch das Kopfteil (3) geführt ist.

10. Verfahren zur Fixierung einer Verbindungsvorrichtung nach einem der Ansprüche 1 bis 8,
**dadurch gekennzeichnet,**
**dass** das Stiftelement (4) mittels eines Laserschweißverfahrens an dem Schaft des Gehäuses (2) unlösbar fixiert wird.

## Claims

1. A connecting device (1) for surgical instruments (100), comprising:
a housing (2) with a shaft;
a head part; and
a head part coupling (10) which is arranged on the shaft of the housing (2) and is designed for mechanically coupling the head part (3), wherein the head part (3) is arranged at a distal end of the shaft and wherein an accessory (7) can be passed through the head part (3);
wherein the head part coupling (10) has a pin element (4) which is designed in such manner that the head part (3) can be fixed immovably to the shaft of the housing (2),
**characterised in that**
the pin element (4) is non-detachably fixed to the shaft of the housing (2) via a weld seam.

2. The connecting device (1) according to claim 1,
**characterised in**
**that** the pin element (4) engages through a bore (5) through the housing (2) and engages into a moulded portion (6) in the head part (3).

3. The connecting device (1) according to claim 1 or 2, **characterised in**
**that** the pin element (4) connects the head part (3) to the housing (2) in a form-fitting manner.

4. The connecting device (1) according to one of the preceding claims,
**characterised in**
**that** the housing (2) and/or the head part (3) is tubular.

5. The connecting device (1) according to one of the preceding claims,
**characterised in**
**that** a monopolar and/or bipolar operable accessory (7) can be passed through the head part (3).

6. The connecting device (1) according to one of the preceding claims,
**characterised in**
**that** the pin element (4) is aligned transversely to a longitudinal axis of the housing (2).

7. The connecting device (1) according to one of the preceding claims,
**characterised in**
**that** the head part (3) contains ceramic or is made of ceramic.

8. The connecting device (1) according to one of the preceding claims,
**characterised in**
**that** said connecting device is designed for a resectoscope.

9. A surgical instrument (100), in particular for high-frequency surgery, comprising:
a connecting device (1) according to one of claims 1 to 8; and
an accessory (7), in particular a monopolar and/or bipolar operable accessory (7),
which is guided through the head part (3).

10. A method for fixing a connecting device according to one of claims 1 to 8,
**characterised in**
**that** the pin element (4) is non-detachably fixed to the shaft of the housing (2) by means of a laser welding process.

## Revendications

1. Dispositif de raccordement (1) pour instruments chirurgicaux (100), comprenant:
un boîtier (2) avec une tige;
une tête; et
un couplage de tête (10) disposé sur la tige du boîtier (2) et conçu pour coupler mécaniquement la tête (3), dans lequel la tête (3) est disposée à une extrémité distale de la tige et dans lequel il est possible de faire passer un accessoire (7) à travers la tête (3);
dans lequel l'accouplement de la tête (10) présente un élément de broche (4) conçu de telle sorte que la tête (3) peut être montée de manière fixe sur la tige du boîtier (2),
**caractérisé en ce que**
l'élément de broche (4) est monté de manière fixe sur la tige du boîtier (2) par un cordon de soudure.

2. Dispositif de raccordement (1) selon la revendication 1,
caractérisé
que l'élément de broche (4) passe à travers un alésage (5) à travers le boîtier (2) et s'engage dans un logement (6) dans la partie de tête (3).

3. Dispositif de raccordement (1) selon la revendication 1 ou 2, **caractérisé**
**en ce que** l'élément de broche (4) relie par complémentarité de forme la tête (3) au boîtier (2).

4. Dispositif de raccordement (1) selon n'importe laquelle des revendications précédentes,
**caractérisé**
**en ce que** le boîtier (2) et/ou la tête (3) ont une forme tubulaire.

5. Dispositif de raccordement (1) selon n'importe laquelle des revendications précédentes,
**caractérisé**
**en ce qu'**un accessoire (7) pouvant fonctionner de manière monopolaire et/ou bipolaire peut être passé à travers la tête (3).

6. Dispositif de raccordement (1) selon n'importe laquelle des revendications précédentes,
**caractérisé**
**en ce que** l'élément de broche (4) est orienté transversalement par rapport à un axe longitudinal du boîtier (2).

7. Dispositif de raccordement (1) selon n'importe laquelle des revendications précédentes,
**caractérisé**
**en ce que** la tête (3) contient de la céramique ou est réalisée en céramique.

8. Dispositif de raccordement (1) selon n'importe laquelle des revendications précédentes,
**caractérisé**
**en ce que** celle-ci est conçue pour un résectoscope.

9. Instrument chirurgical (100), en particulier pour la chirurgie à haute fréquence, comprenant :
un dispositif de raccordement (1) selon n'importe laquelle des revendications 1 à 8; et
un accessoire (7), en particulier un accessoire (7) pouvant fonctionner de manière monopolaire et/ou bipolaire, qui est guidé à travers la partie de tête (3).

10. Procédé de fixation d'un dispositif de raccordement selon n'importe laquelle des revendications 1 à 8,
**caractérisé**
**en ce que** l'élément de broche (4) est monté de manière fixe sur la tige du boîtier (2) au moyen d'un procédé de soudage au laser.
